# FASCICULE DE BREVET EUROPEEN

(11) **EP 4 076 753 B1**
(45) Date de publication et mention de la délivrance du brevet: **09.08.2023**
(21) Numéro de dépôt: 20848709.0
(22) Date de dépôt: 17.12.2020
(51) Int. Cl.: B01L 3/00, B01L 1/02, A61B 50/30, A61L 2/00, B65B 17/02, B65B 55/00, G21F 7/005, A61L 2/26, A61B 50/00, B65B 5/04, B65B 7/28, B65B 39/08, B65B 55/02

(54) **CONTENEUR À ÉTANCHÉITÉ RENFORCÉE**
BEHÄLTER, DER VERSTÄRKTE DICHTEIGENSCHAFTEN AUFWEIST
CONTAINER HAVING REINFORCED SEALING PROPERTIES

(30) Priorité: 20.12.2019 FR 1915253
(43) Date de publication de la demande: 26.10.2022
(73) Titulaire: ABC Transfer, 37000 Tours (FR)
(72) Inventeur: FELIX, Julien, 41100 Vendome (FR); SCHNEIDER, Jean-Luc, 41100 Saint Firmin des Pres (FR); GIRARD, Thierry, 75116 Paris (FR)
(74) Mandataire: IP Trust
(86) Numéro de dépôt international: PCT/FR2020/052499
(87) Numéro de publication internationale: WO 2021/123637

(56) Documents cités:
- EP-A1- 3 147 026
- WO-A1-2014/076208
- FR-A1- 3 010 118

## Description

### DOMAINE TECHNIQUE DE L'INVENTION

L'invention concerne le domaine des conteneurs destinés à être raccordés de manière étanche à des enceintes stériles afin de permettre la mise en communication de leurs volumes respectifs sans contact avec ledit milieu extérieur.

L'invention concerne plus particulièrement un conteneur étanche comprenant une bride délimitant une ouverture de passage d'axe axial dans le conteneur, une porte montée mobile sur la bride et un premier joint d'étanchéité annulaire situé entre la bride et la porte, la bride, la porte et le premier joint d'étanchéité formant un dispositif de connexion étanche apte à être raccordé à un dispositif de connexion complémentaire d'une enceinte stérile par des moyens de raccordement à baïonnette en vue de permettre un communication stérile entre le conteneur étanche et l'enceinte stérile.

Le conteneur étanche selon l'invention est destiné notamment, mais non exclusivement au transfert de produits dangereux comme certains produits pharmaceutiques, biotechnologiques, biologiques, chimiques ou radioactifs, du transfert de composants tels que bouchons, flacons, pistons, seringues, etc., au transfert de dispositifs de contrôle environnementaux tels que des supports de milieu de culture, des compteurs particulaires, etc., du transfert de systèmes de nettoyage, du transfert de liquides, de poudres, d'outils, du transfert de déchets vers l'extérieur de l'enceinte et/ou du transfert de tout élément nécessaire à la production ou à la maintenance de la ligne de production.

### ETAT DE LA TECHNIQUE

De manière connu, le raccordement d'un conteneur étanche à une enceinte stérile pour le transfert de produits, sans rupture de la connexion et de l'étanchéité, est réalisé à l'aide de deux dispositifs de connexion, l'un équipant le conteneur, l'autre équipant l'enceinte. Chaque dispositif de connexion comporte une bride délimitant respectivement l'ouverture de passage dans le volume intérieur du conteneur ou de l'enceinte, chacune des ouvertures de passage étant obturées par une porte. La bride et la porte du conteneur sont aptes à être raccordées respectivement à la bride et à la porte de l'enceinte par une liaison à baïonnettes et désolidarisées l'une de l'autre sous l'action d'un mouvement de rotation de la bride et porte associée du conteneur par rapport à la bride et porte de l'enceinte sur lesquelles elles sont accolées. FR3010118A1 fait partie de l'état de la technique.

Les dispositifs de jonction donnent dans l'ensemble satisfaction d'un point de vue étanchéité. Cependant, il ne peut être exclu que ces dispositifs soient endommagés au cours de leur transport, de leur stockage et/ou de leur manipulation et donc que l'étanchéité des conteneurs ne soit altérée.

L'invention vise à remédier à ce problème en proposant un conteneur étanche raccordable à une enceinte stérile permettant de renforcer l'étanchéité du conteneur tout en garantissant que le conteneur n'a pas subi une altération d'un point de vue étanchéité au cours de son transport et/ou stockage.

### OBJET DE L'INVENTION

A cet effet, l'invention propose un conteneur étanche comprenant une bride délimitant une ouverture de passage d'axe axial dans le conteneur, une porte montée mobile sur la bride et un premier joint d'étanchéité annulaire situé entre la bride et la porte, la bride, la porte et le premier joint d'étanchéité formant un dispositif de connexion étanche apte à être raccordé à un dispositif de connexion complémentaire d'une enceinte stérile par des moyens de raccordement à baïonnette en vue de permettre un communication stérile entre le conteneur étanche et l'enceinte stérile. Le conteneur est remarquable en ce qu'il comporte un capot de protection du dispositif de connexion clipsé en force sur le conteneur et des moyens de pression de contact arrangés entre le capot de protection et le dispositif de connexion de manière à exercer une pression de contact sur le dispositif de connexion suivant la direction axiale proportionnelle à la force de clipsage.

Avantageusement, les moyens de pression de contact sont arrangés pour exercer une pression de contact sur la porte.

Il peut être prévu également que les moyens de pression de contact soient arrangés pour exercer une pression de contact sur le premier joint d'étanchéité seul ou également sur la porte.

Avantageusement, le capot de protection comporte une surface interne pourvue d'un bosselage annulaire formant les moyens de pression. Le bosselage annulaire peut être continu ou discontinu.

Avantageusement, les moyens de pression sont formés par un deuxième joint d'étanchéité annulaire intercalé entre le capot de protection et le dispositif de connexion.

Avantageusement, le conteneur présente un corps de conteneur formé par un film souple fixé sur la bride, ladite bride étant constituée de deux pièces distinctes l'une de l'autre, formant respectivement un manchon externe et un manchon interne, le film souple étant maintenu en prise entre le manchon externe et le manchon interne.

Avantageusement, le conteneur comporte une bague de serrage du manchon externe sur le manchon interne arrangé pour enserrer les parties du manchon externe et du manchon interne entre lesquelles le film souple est interposé.

Avantageusement, le capot de protection comporte des languettes flexibles s'étendant en direction de la bague de serrage et en prise avec ce dernier de manière irréversible.

Avantageusement, le conteneur comporte un troisième joint d'étanchéité annulaire logé dans une gorge périphérique interne du manchon externe, le film souple s'étendant entre le joint annulaire et le manchon interne.

Avantageusement, le manchon externe comporte une jupe inférieure comprenant des fentes longitudinales s'étendant depuis la bordure d'extrémité inférieure du manchon externe et équiréparties sur la périphérie dudit manchon de sorte à former des languettes élastiques aptes à exercer un effort de serrage sur le manchon interne.

Avantageusement, le film souple et le dispositif de connexion sont réalisés en un matériau bactériostatique.

### BREVE DESCRIPTION DES FIGURES

D'autres caractéristiques et avantages de l'invention ressortiront de la description détaillée de l'invention qui va suivre en référence aux figures annexées et dans lesquelles :

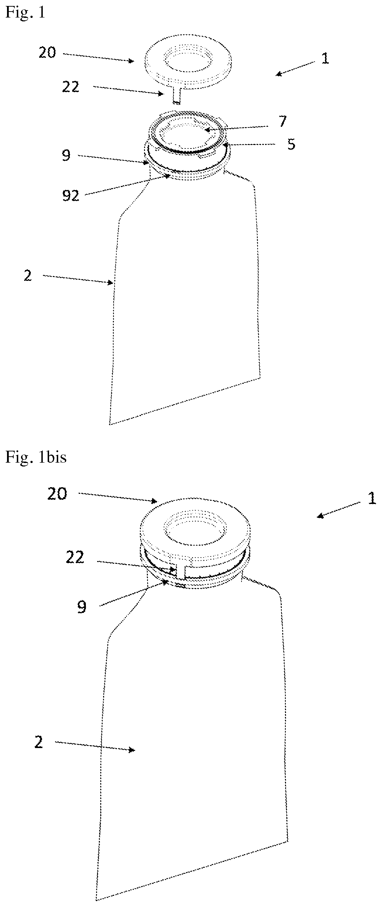

- La figure 1 représente une vue schématique en perspective d'un conteneur selon l'invention, le capot de protection étant représenté non monté ;
- La figure 1bis représente une vue du conteneur de la figure 1 équipé du capot de protection ;
- La figure 2 représente une vue en coupe du conteneur de la figure 1bis ;
- La figure 3 représente une vue de détail du conteneur de la figure 2 ;
- La figure 4 représente une vue partielle du manchon externe 5 du dispositif de connexion du conteneur de la figure 2 ;
- La figure 5 représente une autre vue de détail du conteneur de la figure 2 :
- La figure 6 représente une vue de dessous du capot de protection du conteneur ;
- La figure 7 représente une autre vue de détail du conteneur de la figure 2 ;

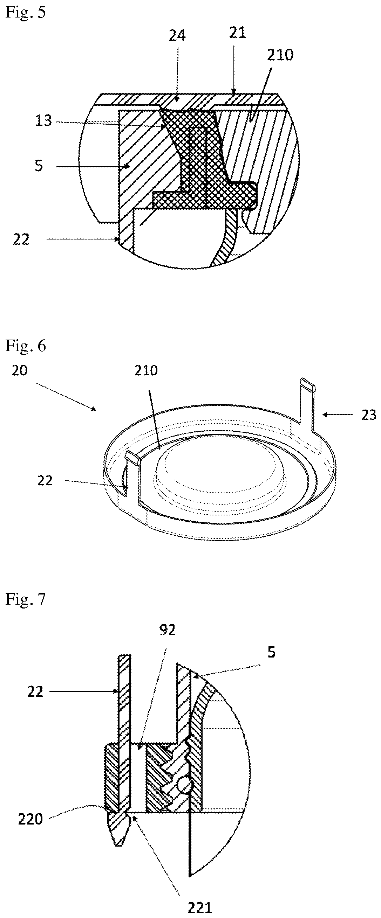

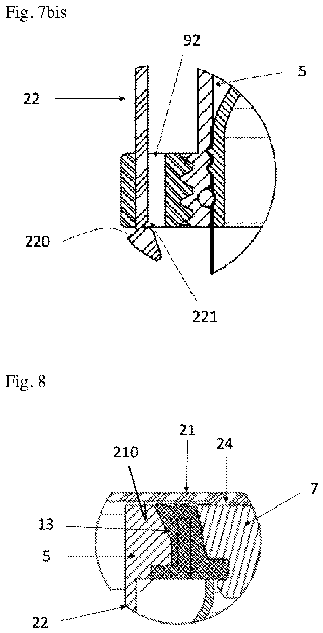

- La figure 7bis reprend la vue de détail de la figure 7, une languette du capot de protection étant représentée déformée ;
- La figure 8 représente une vue de détail d'une variante de réalisation du conteneur selon l'invention.

### DESCRIPTION DETAILLEE DE L'INVENTION

En relation avec les figures 1 à 6, il est décrit un conteneur 1 selon un exemple de réalisation de l'invention. Le conteneur 1 illustré comprend un film souple 2 arrangé pour définir un volume intérieur 3 et une ouverture de passage, définissant un axial, permettant de mettre en communication le volume intérieur 3 avec l'extérieur du film. Le conteneur comporte également un dispositif de connexion étanche lequel est arrangé pour permettre la connexion du film souple 2 à un dispositif de connexion complémentaire d'une enceinte stérile. La connexion est réalisée de manière à assurer une communication stérile entre le volume intérieur 3 dudit conteneur 1 et celui de l'enceinte.

Le dispositif de connexion comprend une bride sur laquelle le film souple est fixé, ladite bride délimitant l'ouverture de passage du film souple. Il comprend en outre une porte 7 amovible fixée sur la bride de façon à obturer l'ouverture de passage. Avantageusement, la bride et la porte 7 sont fixées l'une à l'autre au moyen d'encoches et d'oreilles portées respectivement par la bride et la porte.

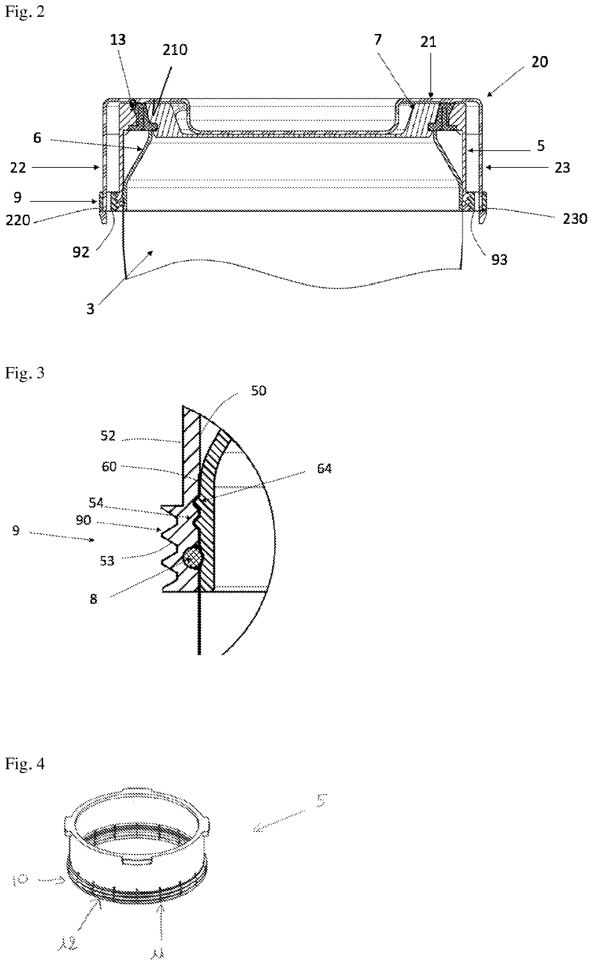

Dans l'exemple illustré, la bride est constituée de deux pièces distinctes l'une de l'autre, une première pièce formant un manchon dit externe et une deuxième pièce formant un manchon dit interne, entre lesquels le film souple s'étend. Le manchon interne 6 comporte une zone crantée 64 adjacente au renfoncement d'appui arrangée pour coopérer avec un crantage complémentaire 54 ménagé sur la face interne 50 du manchon externe 5. Le manchon externe 5 comporte, selon une configuration avantageuse illustrée sur la figure 4, une jupe inférieure 10 comprenant des fentes longitudinales 11 s'étendant depuis la bordure d'extrémité inférieure dudit manchon. Ces fentes longitudinales 11 sont avantageusement équiréparties sur la périphérie dudit manchon. Elles délimitent ainsi des languettes élastiques 12 aptes à exercer un effort de serrage homogène sur l'ensemble de la périphérie extérieure du manchon interne 6 lorsque le manchon externe 5 est monté sur le manchon interne 6. Les languettes élastiques 12 comportent des moyens d'association réciproque avec respectivement des moyens de serrage, décrits ci-après, et le manchon interne 6. Dans l'exemple illustré, les moyens d'association réciproque respectivement avec les moyens de serrage et le manchon interne 6 consistent en un filetage 53 et un crantage 54. Les moyens de serrage se présentent sous la forme d'une bague de serrage 9 externe comprenant un taraudage 90 de forme complémentaire au filetage 53 ménagé sur la face externe 52 du manchon externe 5. Les moyens de serrage sont arrangés pour enserrer les parties du manchon externe 5 et du manchon interne 6 entre lesquelles le film souple 2 est interposé.

Le conteneur 1 comporte en outre un joint d'étanchéité annulaire 8 logé dans une gorge périphérique ménagée sur la face interne 50 du manchon externe 5 et la face externe 60 du manchon interne 6. Il définit un joint d'étanchéité bride/sac. Comme montré sur la figure 3, le film souple 2 est maintenu en prise entre le joint annulaire 8 et le manchon interne 6. Afin d'améliorer la retenue du film souple 2 entre les deux manchons constitutifs de la bride, le manchon interne 6 comporte une face externe pourvue d'un renfoncement d'appui situé en vis-à-vis de la gorge périphérique du manchon externe 5. Ainsi, lors du serrage du manchon externe 5 sur le manchon interne 6, le joint annulaire 8 écrase le film souple 2 au niveau du renfoncement d'appui, assurant une mise en prise améliorée du film souple 2.

Le conteneur 1 comporte en outre un joint d'étanchéité annulaire 13 lequel est porté par la bride. Il définit un joint d'étanchéité porte/bride. Lorsque la porte est en position d'obturation de l'ouverture de passage, le joint annulaire 13 est logé dans l'espace délimité par le manchon externe 5, le manchon interne 6 et la porte 7. Avantageusement, le joint annulaire 13 présente une section de forme conjuguée à l'espace dans lequel il est logé. Dans le mode de réalisation illustré, le joint annulaire 13 présente une section en forme de L.

Selon l'invention, le conteneur comporte un capot de protection 20 du dispositif de connexion clipsé en force sur la bride 5, 6. Dans le mode de réalisation illustré, le capot de protection 20 comporte une partie couvrante 21 placée en vis-à-vis de la face avant du conteneur, à la périphérie extérieure de laquelle s'étendent deux languettes 22, 23 en direction de la bague de serrage 9. Par face avant, on désigne la face du conteneur par rapport à son raccordement avec l'enceinte. En d'autres termes, il s'agit de la face du conteneur amenée en vis-à-vis de l'enceinte pour le raccordement dudit conteneur à l'enceinte.

Chaque languette 22, 23 est pourvue au niveau de son extrémité libre d'un épaulement 220, 230 formé par un renflement de matière qui s'étend, dans l'exemple illustré, radialement du côté opposé de la partie couvrante 21. Les languettes 22, 23 s'étendent, avantageusement sensiblement parallèlement à l'axe axial en direction de la bague de serrage 9 et traversent respectivement une lumière 92, 93 associée ménagée dans ladite bague de serrage 9 de sorte que les épaulements 220, 230 sont situés sous la bague de serrage 9. Ainsi, comme représenté sur la figure 2, le clipsage du capot de protection 20 consiste à mettre en prise les épaulements 220, 230 des languettes 22, 23 sur le rebord d'appui arrière de la bague de serrage 9 délimitant les lumières.

Dans le mode de réalisation illustré, le capot de protection 20 comporte deux languettes 22, 23 de clipsage situées diamétralement opposées l'une de l'autre. Il s'agit d'un exemple de réalisation, le nombre et la répartition des languettes pouvant varier suivant les dimensions de l'ouverture du conteneur.

Le capot de protection 20 comporte en outre des moyens de pression de contact arrangés entre le capot de protection 20 et le dispositif de connexion de manière à exercer une pression de contact sur le premier joint d'étanchéité 13 suivant la direction axiale proportionnelle à la force de clipsage. Dans l'exemple illustré, les moyens de pression sont formés par un bosselage annulaire 24 de la face interne 210 de la partie couvrante 21. Le bosselage annulaire 24 est ainsi aménagé pour être placé en vis-à-vis du joint d'étanchéité porte/bride 13 lorsque le capot de protection 20 est placé sur le conteneur. Dans ce mode de réalisation, les moyens de pression sont formés d'un seul tenant avec le capot de protection 20. Selon une variante de réalisation, les moyens de pression peuvent constitué par une pièce rapportée, comme par exemple un joint d'étanchéité, disposé entre le capot de protection 20 et le joint d'étanchéité porte/bride 13. Dans le mode de réalisation décrit, le bosselage annulaire est continu. Selon une variante de réalisation, il peut être prévu un bosselage discontinu formant des points de pression distincts.

Avantageusement, les languettes 22, 23 sont arrangées pour que le retrait du capot de protection 20 puisse être réalisé par déformation de celles-ci. Pour ce faire, elles comportent chacune une encoche de forme en V aménagée en opposé à l'épaulement 220, 230. Les figures 7 et 7bis montrent une vue de détail d'une languette ainsi arrangée, seule la languette 22 et son encoche 221 étant représentées Il pourrait être également prévu, selon une variante de réalisation, que les languettes 22, 23 soient sécables au niveau des encoches. Ainsi, selon le matériau employé, la fragilisation obtenue via les encoches permet soit une déformation localisée des languettes 22, 23, soit de casser les languettes au niveau de l'encoche.

Du fait de l'arrangement des éléments le composant, le dispositif de connexion est assemblé par simple clipsage des éléments en eux. Pour réaliser le conteneur 1, la partie du film souple, située du côté de l'ouverture de passage, sera disposée de façon à être placée entre le manchon externe 5 et le manchon interne 6. Le serrage sera effectif par serrage de la bague 9 sur le manchon interne 6, entrainant le resserrement des languettes 12 contre le manchon interne 6. Le film souple 2 pourra être désassemblé en dévissant la bague de serrage 9 et en déclipsant le manchon externe 5 du manchon interne 6. Le désassemblage du film souple ne pourra être cependant réalisé qu'après retrait du capot de protection 20.

Le film souple 2 et le dispositif de connexion sont avantageusement réalisés en un matériau bactériostatique. Les joints d'étanchéité 8 et 13 sont réalisés en un matériau silicone ou autres matériaux minimisant les phénomènes de rémanence. Il peut être prévu également que les joints d'étanchéité soient réalisés en un matériau bactériostatique.

Dans le mode de réalisation venant d'être décrit, les moyens de pression de contact sont arrangés pour exercer une pression de contact sur le joint d'étanchéité. Selon une variante de réalisation illustré sur la figure 8, il peut être prévu que les moyens de pression de contact soient arrangés pour exercer une pression de contact sur la porte 7. Comme décrit précédemment, les moyens de pression peuvent être formés par un bosselage annulaire 24 ; continu ou discontinu, ménagé au niveau de la face interne 210 de la partie couvrante 21 et aménagé pour être placé en vis-à-vis de la porte 7 lorsque le capot de protection 20 est placé sur le conteneur. Ils peuvent être également portés par une pièce rapportée (joint d'étanchéité ou autre) disposée entre le capot de protection 20 et la porte 7. Dans le mode de réalisation décrit, le bosselage annulaire est continu.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Conteneur (1) étanche comprenant une bride (5, 6) délimitant une ouverture de passage d'axe axial dans le conteneur, une porte (7) montée mobile sur la bride (5, 6) et un premier joint d'étanchéité annulaire (13) situé entre la bride (5, 6) et la porte (7), la bride, la porte et le premier joint d'étanchéité formant un dispositif de connexion étanche apte à être raccordé à un dispositif de connexion complémentaire d'une enceinte stérile par des moyens de raccordement à baïonnette en vue de permettre un communication stérile entre le conteneur étanche et l'enceinte stérile,
**caractérisé en ce que** le conteneur (1) comporte :
- un capot de protection (20) du dispositif de connexion clipsé en force sur le conteneur,
- des moyens de pression de contact arrangés entre le capot de protection (20) et le dispositif de connexion de manière à exercer une pression de contact sur le dispositif de connexion suivant la direction axiale proportionnelle à la force de clipsage.

2. Conteneur (1) étanche selon la revendication 1, **caractérisé en ce que** les moyens de pression de contact sont arrangés pour exercer une pression de contact sur la porte.

3. Conteneur (1) étanche selon la revendication 1 ou la revendication 2, **caractérisé en ce que** les moyens de pression de contact sont arrangés pour exercer une pression de contact sur le premier joint d'étanchéité (13).

4. Conteneur (1) étanche selon l'un quelconque des revendications 1 à 3, **caractérisé en ce que** le capot de protection (20) comporte une surface interne pourvue d'un bosselage annulaire formant les moyens de pression.

5. Conteneur (1) étanche selon la revendication précédente, **caractérisé en ce que** le bosselage annulaire est discontinu.

6. Conteneur (1) étanche selon l'un quelconque des revendications 1 à 3, **caractérisé en ce que** les moyens de pression sont formés par un deuxième joint d'étanchéité annulaire intercalé entre le capot de protection (20) et le dispositif de connexion.

7. Conteneur (1) étanche selon l'un quelconque des revendications précédentes, **caractérisé en ce qu'**il présente un corps de conteneur formé par un film souple (2) fixé sur la bride, ladite bride étant constituée de deux pièces distinctes l'une de l'autre, formant respectivement un manchon externe (5) et un manchon interne (6), le film souple (2) étant maintenu en prise entre le manchon externe (5) et le manchon interne (6).

8. Conteneur (1) étanche selon la revendication précédente, **caractérisé en ce qu'**il comporte une bague de serrage (9) du manchon externe (5) sur le manchon interne (6) arrangé pour enserrer les parties du manchon externe (5) et du manchon interne (6) entre lesquelles le film souple est interposé.

9. Conteneur (1) étanche selon la revendication précédente, **caractérisé en ce que** le capot de protection (20) comporte des languettes (22, 23) flexibles s'étendant en direction de la bague de serrage (9) et en prise avec ce dernier de manière irréversible.

10. Conteneur (1) étanche selon la revendication 5 ou la revendication 6, **caractérisé en ce qu'**il comporte un troisième joint d'étanchéité annulaire (8) logé dans une gorge périphérique interne du manchon externe (5), le film souple (2) s'étendant entre le joint annulaire (8) et le manchon interne (6).

11. Conteneur (1) étanche selon l'une quelconque des revendications précédentes, **caractérisé en ce que** le manchon externe (5) comporte une jupe inférieure (10) comprenant des fentes longitudinales (11) s'étendant depuis la bordure d'extrémité inférieure du manchon externe (5) et équiréparties sur la périphérie dudit manchon de sorte à former des languettes élastiques (12) aptes à exercer un effort de serrage sur le manchon interne (6).

12. Conteneur (1) étanche selon l'une quelconque des revendications précédentes, **caractérisé en ce que** le film souple (2) et le dispositif de connexion sont réalisés en un matériau bactériostatique.

## Patentansprüche

1. Dichter Behälter (1) umfassend einen Flansch (5, 6), der eine Durchgangsöffnung mit axialer Achse in dem Behälter begrenzt, eine Tür (7), die beweglich an dem Flansch (5, 6) angebracht ist, und eine erste ringförmige Dichtung (13), die sich zwischen dem Flansch (5, 6) und der Tür (7) befindet, wobei der Flansch, die Tür und die erste Dichtung eine dichte Verbindungsvorrichtung bilden, die geeignet ist, an eine ergänzende Verbindungsvorrichtung einer sterilen Kammer durch Bajonettanschlussmittel angeschlossen zu werden, um eine sterile Kommunikation zwischen dem dichten Behälter und der sterilen Kammer zu erlauben,
**dadurch gekennzeichnet, dass** der Behälter (1) aufweist:
- eine Schutzkappe (20) der Verbindungsvorrichtung, die fest auf den Behälter geklipst wird,
- Anpressdruckmittel, die zwischen der Schutzkappe (20) und der Verbindungsvorrichtung so angeordnet sind, dass sie einen Anpressdruck auf die Verbindungsvorrichtung entlang der axialen Richtung proportional zu der Klipskraft ausüben.

2. Dichter Behälter (1) nach Anspruch 1, **dadurch gekennzeichnet, dass** die Anpressdruckmittel so angeordnet sind, dass sie einen Anpressdruck auf die Tür ausüben.

3. Dichter Behälter (1) nach Anspruch 1 oder 2,
**dadurch gekennzeichnet, dass** die Anpressdruckmittel so angeordnet sind, dass sie einen Anpressdruck auf die erste Dichtung (13) ausüben.

4. Dichter Behälter (1) nach einem der Ansprüche 1 bis 3,
**dadurch gekennzeichnet, dass** die Schutzkappe (20) eine Innenoberfläche aufweist, die mit einer ringförmigen Erhebung versehen ist, die die Druckmittel bildet.

5. Dichter Behälter (1) nach dem vorstehenden Anspruch,
**dadurch gekennzeichnet, dass** die ringförmigen Erhebungen unterbrochen sind.

6. Dichter Behälter (1) nach einem der Ansprüche 1 bis 3,
**dadurch gekennzeichnet, dass** die Druckmittel durch eine zweite ringförmige Dichtung gebildet werden, die zwischen der Schutzkappe (20) und der Verbindungsvorrichtung eingesetzt ist.

7. Dichter Behälter (1) nach einem der vorstehenden Ansprüche,
**dadurch gekennzeichnet, dass** er einen Behälterkörper vorweist, der durch eine flexible Folie (2) gebildet wird, die an dem Flansch befestigt ist, wobei der Flansch aus zwei voneinander verschiedenen Teilen besteht, die jeweils eine äußere Hülse (5) und eine innere Hülse (6) bilden, wobei die flexible Folie (2) zwischen der äußeren Hülse (5) und der inneren Hülse (6) in Eingriff gehalten wird.

8. Dichter Behälter (1) nach dem vorstehenden Anspruch,
**dadurch gekennzeichnet, dass** er einen Spannring (9) der äußeren Hülse (5) auf der inneren Hülse (6) aufweist, der so angeordnet ist, dass er die Teile der äußeren Hülse (5) und der inneren Hülse (6), zwischen denen die flexible Folie liegt, einspannt.

9. Dichter Behälter (1) nach dem vorstehenden Anspruch,
**dadurch gekennzeichnet, dass** die Schutzkappe (20) flexible Laschen (22, 23) aufweist, die sich in Richtung des Spannrings (9) erstrecken und irreversibel mit diesem in Eingriff gehalten werden.

10. Dichter Behälter (1) nach Anspruch 5 oder 6,
**dadurch gekennzeichnet, dass** er eine dritte ringförmige Dichtung (8) aufweist, die in einer inneren Umfangsnut der äußeren Hülse (5) aufgenommen ist, wobei sich die flexible Folie (2) zwischen der ringförmigen Dichtung (8) und der inneren Hülse (6) erstreckt.

11. Dichter Behälter (1) nach einem der vorstehenden Ansprüche,
**dadurch gekennzeichnet, dass** die äußere Hülse (5) einen unteren Mantel (10) aufweist, umfassend Längsschlitze (11), die sich von der unteren Endkante der äußeren Hülse (5) aus erstrecken und gleichmäßig über den Umfang der Hülse verteilt sind, so dass sie elastische Laschen (12) bilden, die geeignet sind, eine Spannkraft auf die innere Hülse (6) auszuüben.

12. Dichter Behälter (1) nach einem der vorstehenden Ansprüche, **dadurch gekennzeichnet, dass** die flexible Folie (2) und die Verbindungsvorrichtung aus einem bakteriostatischen Material hergestellt sind.

## Claims

1. A sealed container (1) comprising a flange (5, 6) delimiting a passage opening having an axial axis in the container, a door (7) movably mounted on the flange (5, 6) and a first annular seal (13) located between the flange (5, 6) and the door (7), the flange, the door and the first seal forming a sealed connection device suitable for being connected to a complementary connection device of a sterile enclosure by bayonet connection means with a view to enabling sterile communication between the sealed container and the sterile enclosure,
**characterized in that** the container (1) includes:
- a protective cover (20) of the connection device forcibly snap-fitted onto the container,
- contact pressure means arranged between the protective cover (20) and the connection device so as to apply a contact pressure to the connection device in the axial direction which is proportional to the snap-fitting force.

2. The sealed container (1) according to claim 1, **characterized in that** the contact pressure means are arranged so as to apply a contact pressure to the door.

3. The sealed container (1) according to claim 1 or claim 2,
**characterized in that** the contact pressure means are arranged so as to apply a contact pressure to the first seal (13).

4. The sealed container (1) according to any of claims 1 to 3,
**characterized in that** the protective cover (20) has an inner surface provided with an annular embossing forming the pressure means.

5. The sealed container (1) according to the preceding claim, **characterized in that** the annular embossing is intermittent.

6. The sealed container (1) according to any of claims 1 to 3,
**characterized in that** the pressure means are formed by a second annular seal inserted between the protective cover (20) and the connection device.

7. The sealed container (1) according to any of the preceding claims, **characterized in that** it has a container body formed by a flexible film (2) attached to the flange, said flange consisting of two parts separate from one another, respectively forming an outer sleeve (5) and an inner sleeve (6), the flexible film (2) being held in engagement between the outer sleeve (5) and the inner sleeve (6).

8. The sealed container (1) according to the preceding claim,
**characterized in that** it has a ring (9) for clamping the outer sleeve (5) on the inner sleeve (6), which ring is arranged so as to clamp the portions of the outer sleeve (5) and the inner sleeve (6) between which the flexible film is positioned.

9. The sealed container (1) according to the preceding claim,
**characterized in that** the protective cover (20) has flexible tabs (22, 23) extending in the direction of the clamping ring (9) and engaging irreversibly therewith.

10. The sealed container (1) according to claim 5 or claim 6,
**characterized in that** it has a third annular seal (8) accommodated in an inner peripheral groove of the outer sleeve (5), the flexible film (2) extending between the annular seal (8) and the inner sleeve (6).

11. The sealed container (1) according to any of the preceding claims, **characterized in that** the outer sleeve (5) comprises a lower skirt (10) having longitudinal slots (11) extending from the lower end edge of the outer sleeve (5) and evenly distributed over the periphery of said sleeve so as to form elastic tabs (12) suitable for applying a clamping force to the inner sleeve (6).

12. The sealed container (1) according to any of the preceding claims, **characterized in that** the flexible film (2) and the connection device are made of a bacteriostatic material.
